# EUROPEAN PATENT APPLICATION

(11) **EP 1 378 501 A1**
(43) Date of publication of application: **07.01.2004**
(21) Application number: 03101541.5
(22) Date of filing: 27.05.2003
(51) Int. Cl.: C07B 41/02, C07C 67/31, C07C 69/675, C07C 29/145, C07B 53/00

(54) **Diastereoselective hydrogenation of beta-hydroxyketones**

(30) Priority: 02.07.2002 EP 20140976
(71) Applicant: Solvias AG, 4057 Basel (CH)
(72) Inventor: STUDER, Martin, 4054, Basel (CH); BURKHARDT, Stephan, 4460, Gelterkinden (CH); NETTEKOVEN, Ulrike, 4055, Basel (CH)
(74) Representative: Maué, Paul Georg

(57) **Abstract**

The invention relates to a novel process of diastereoselective hydrogenation of 1,3-hydroxyketones of formula (I) 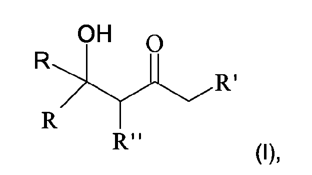 wherein R, R' and R" are as defined in claim 1
which is carried out in a solvent in the presence of a magnesium salt, a heterogeneous platinum catalyst and optionally an oxidant.

## Description

This invention relates to a novel process of diastereoselective hydrogenation of 1,3-hydroxyketones.

Stereoselective preparation of the 1,3-diol function has great utility in organic chemistry due to the occurrence of such a group in natural products. Besides enzymatic routes, there are also transition-metal-catalysed hydrogenation reactions described. For example, in *Eur. J. Org. Chem.* **1999,** 1787-1793, the enantioselective homogeneous catalysis using rhodium- or ruthenium-bisphosphane complexes are described. However, the different proposed routes are not fully satisfactory.

It is therefore an object of the instant invention to provide for a hydrogenation process using a simple, commercially available heterogeneous catalyst which can be filtered off after the reaction. It is a further object that the process has a good selectivity and that the hydrogenation proceeds fast. It is also an object to provide for a process not using expensive compounds. A further object is a process which does not necessarily require low temperature thus avoiding cooling equipment. An even further object is a process whereby the waste problems are minimised.

Surprisingly, it has now been found that the addition of magnesium salts to a heterogeneous catalytic system based on platinum catalysts significantlyimproves the diastereoselective hydrogenation of 1,3-hydroxyketones. A further surprising improvement, especially with regard to the overall conversion and the diastereomeric ratio is observed when a catalytic amount of an oxidant such as H₂O₂ is added to the reaction mixture.

In general, the beneficial effects of the inventive reaction are observed for a large structural variety of 1,3-hydroxyketones and in general at least an increase in the conversion is observed by the addition of a magnesium salt and the optional addition of a catalytic amount of an oxidant. Such increase is in particular also observed for acyl derivatives of 1,3-hydroxyketones and 1,3-hydroxyketones which are twice substituted in the 2-position.

The invention especially relates to a process, wherein a compound of formula (I) 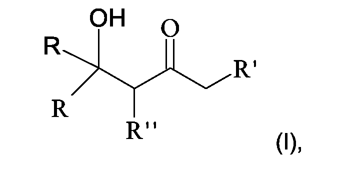 wherein R, R' and R" are independently of each other a radical being compatible with the reaction conditions,
except compounds wherein
a) one R is H and the other R is -CH₂CN, R" is H and R' is -C(=O)OR_{b} and R_{b} is a H or a carboxy-protecting group;
b) one R is H and the other R is -CH₂C(=O)NR*R**, R" is H and R' is -C(=O)OR_{b} and R* and R** are independently of each other H or an amide-protecting group and R_{b} is H or a carboxy-protecting group;
c) one R is H and the other R is -CH₂C(=O)OR_{b}, R" is H and R' is -CH₂-N₃ and R_{b} is H or a carboxy-protecting group; and
d) one R is H and the other R is -CH₂C(=O)OR_{b}, R" is H and R' is -CH₂-R_{d} and R_{b} is H or a carboxy-protecting group and R_{d} is halogen;
is reduced with hydrogen to the corresponding diol, which is predominantly in the form of the syn-diol, in the presence of a magnesium salt and a heterogeneous platinum catalyst in a solvent.

Unless otherwise indicated, halogen is preferably fluorine, chlorine, bromine or iodine.

Suitable compatible radicals R, whereby the two radicals R must be different from each other, are for example H, alkyl, haloalkyl, hydroxyalkyl, alkoxyalkyl, haloalkoxy, cycloalkyl, cycloalkoxy, cycloalkyl-alkyl, cycloalkylalkoxy, aryl, aryloxy, aralkyl, aralkoxy, -CH₂CO₂R₄, -CHR₄CO₂R₅, -CO₂R₄, -CH₂C(O)NH₂, -CH₂C(O)NHR₄, -CH₂C(O)NR₄R_{5,} -CHR₄C(O)NH₂, -CHR₄C(O)NHR₅ -CHR₄C(O)NR₅R₆, -C(O)-NH₂, -C(O)-NHR₄ and -C(O)-NR₄R₅, wherein R₄, R₅ and R₆ are independently from each other alkyl, cycloalkyl, cycloalkylalkyl, phenyl or benzyl; to the extent possible said radicals may be branched or unbranched and may be unsubstituted or substituted. Preferred radicals R are H, alkyl (most preferred C₁-C₁₂-alkyl), aryl (most preferred phenyl or naphthyl), -CH₂CO₂R₄, -CHR₄CO₂R₅, -CO₂R₄, -CH₂C(O)NH₂, -CH₂C(O)NHR₄, -CH₂C(O)NR₄R₅ -CHR₄C(O)NH₂, -CHR₄C(O)NHR₅, -CHR₄C(O)NR₅R₆, -C(O)-NH₂, -C(O)-NHR₄ and -C(O)-NR₄R₅ which independently of each other may be unsubstituted or substituted. Also preferred are compounds where at least one radical R is H or lower alkyl, whereby H is particularly preferred. Suitable substituents are apparent from the given lists of compatible radicals and protecting groups. Preferred substituents are halogen, oxygen, nitrogen, hydroxy and lower alkoxy.

Suitable compatible radicals R' or R" are for example H, alkyl, alkoxy, haloalkyl, hydroxyalkyl, alkoxyalkyl, haloalkoxy, cycloalkyl, cycloalkoxy, cycloalkyl-alkyl, cycloalkylalkoxy, aryl, aryloxy, aralkyl, aralkoxy, halogen, -OH, -OR₄, -OC(O)R₄, -NH-C(O)-R₄, -NR₄-C(O)-R₄, -CO₂R₄, -C(O)-NH₂, -C(O)-NHR₄, and -C(O)-NR₄R₅, wherein R₄ and R₅ are independently from each other alkyl, cycloalkyl, cycloalkylalkyl, phenyl or benzyl; to the extent possible said radicals may be branched or unbranched and may be unsubstituted or substituted. Preferred radicals R' are H, alkyl (most preferred C₁-C₁₂-alkyl), aryl (most preferred phenyl or naphthyl), , -CO₂R₄, -C(O)-NH₂, -C(O)-NHR₄, and -C(O)-NR₄R₅ which may be unsubstituted or substituted. Preferred radicals R" are H, alkyl (most preferred C₁-C₁₂-alkyl), cycloalkyl and aryl (most preferred phenyl or naphthyl) which independently of each other may be unsubstituted or substituted. Suitable substituents are apparent from the given lists of compatible radicals and protecting groups. Preferred substituents are halogen, oxygen, nitrogen, hydroxy and lower alkoxy.

The prefix "lower-" or "lower" indicates that the radical in question contains preferably up to 7 carbon atoms, especially up to 4 carbon atoms. Lower alkyl is therefore preferably C₁-C₇-alkyl, especially C₁-C₄-alkyl, and may be unbranched or branched one or more times, insofar as possible. Cyclic radicals, such as cycloalkyl, have at least 3 carbon atoms, especially from 3 to 7.

Carboxy-protecting groups are especially ester-forming, enzymatically and/or chemically removable protecting groups, preferably enzymatically and/or chemically removable protecting groups, such as heptyl, 2-N-(morpholino)ethyl, cholinyl, methoxyethoxyethyl or methoxyethyl; or those which are primarily chemically removable, e.g. alkyl, such as lower alkyl, especially methyl, ethyl, substituted lower alkyl (except for benzyl and substituted benzyl), such as substituted methyl, especially 9-fluorenylmethyl, methoxymethyl, methoxyethoxymethyl, methylthiomethyl, 2-(trimethylsilyl)ethoxymethyl, benzyloxymethyl, pivaloyloxymethyl, phenylacetoxymethyl, triisopropylsilyl methyl, 1-3-dithianyl-2-methyl, dicyclopropylmethyl, acetonyl, phenacyl, p-bromophenacyl, α-methylphenacyl, p-methoxyphenacyl, desyl, carbamidomethyl, p-azobenzenecarboxamidomethyl, N-phthalimidomethyl or 4-picolyl, 2-substituted ethyl, especially 2-iodo-, 2-bromo- or 2-chloro-ethyl, 2,2,2-trichloroethyl, 2-(trimethylsilyl)ethyl, 2-methylthioethyl, 2-(p-nitrophenylsulfenyl)ethyl, 2-(p-toluenesulfonyl)-ethyl, 2-(2'-pyridyl)ethyl, 2-(p-methoxyphenyl)ethyl, 2-(diphenylphosphino)ethyl, 1-methyl-1-phenylethyl, 2-(4-acetyl-2-nitrophenyl)ethyl or 2-cyanoethyl, tert-butyl, 3-methyl-3-pentyl, 2,4-dimentyl-3-pentyl or ω-chloro-lower alkyl, especially 4-chlorobutyl or 5-choropentyl, cyclopentyl, cyclohexyl, lower alkenyl, especially allyl, methallyl, 2-methylbut-3-en-2-yl, 3-methylbut-2-enyl or 3-buten-1-yl, substituted lower alkenyl, especially 4-(trimethylsilyl)-2-buten-1-yl, cinnamyl or α-methylcinnamyl, lower alkynyl, such as prop-2-ynyl, phenyl, substituted phenyl, especially 2,6-dialkylphenyl, such as 2,6-dimethylphenyl, 2,6-diisoproylphenyl, 2,6-di-tert-butyl-4-methylphenyl, 2,6-di-tert-butyl-4-methoxyphenyl, p-(methylthio)-phenyl or pentafluorophenyl, benzyl, substituted benzyl, especially triphenylmethyl, diphenylmethyl, bis(o-nitrophenyl)methyl, 9-anthrylmethyl, 2-(9,10-dioxo)anthrylmethyl, 5-dibenzosuberyl, 1-pyrenylmethyl, 2-(trifluoromethyl)-6-chromonylmethyl, 2,4,6-trimethylbenzyl, p-bromobenzyl, o-nitrobenzyl, p-nitrobenzyl, p-methoxybenzyl, 2-6-dimethoxybenzyl, 4-(methylsulfinyl)benzyl, 4-sulfobenzyl, 4-azidomethoxybenzyl, 4-{N-[4,4-dimethyl,2,6-dioxocyclohexylidene)-3-methylbutyl]amino}benzyl, piperonyl or p-polymer-benzyl, tetrahydropyranol, tetrahydrofuranyl, or silyl radicals, such as tri-lower alkylsilyl, especially trimethylsilyl, triethylsilyl, tert-butyldiemethylsilyl, isopropyldimethylsilyl or di-tert-butylmethylsilyl, or phenyl-di-lower alkylsilyl, such as phenyldimethylsilyl; alternatively a carboxy group can also be protected in the form of an oxazolyl, 2-alkyl-1,3-oxazolinyl, 4-alkyl-5-oxo-1,3-oxazolodinyl or 2,2-bistrifluoromethyl-4-alkyl-5-oxo-1,3-oxazolodinyl radical.

Amide-protecting groups are especially allyl, tert-butyl, N-methoxy, N-benzoyloxy, N-methylthio, triphenylmethylthio, tert-butyldimethylsilyl, triisopropylsilyl, 4-(methoxymethoxy)phenyl, 2-methoxy-1-naphthyl, 9-fluorenyl, tert-butoxycarbonyl, N-benzyloxycarbonyl, N-methoxy- or N-ethoxy-carbonyl, toluenesulfonyl, N-buten-1-yl, 2-methoxycarbonylvinyl, or especially alkyl, such as lower alkyl, or more especially substituted alkyl, especially benzyl, benzyl substituted by one ore more radicals selected from lower alkoxy, such as methoxy, lower alkanoyloxy, such as acetoxy, lower alkylsulfinyl, such as methylsulfinyl, dicyclopropylmethyl, methoxymethyl, methylthiomethyl and N-benzoyloxymethyl; or bis(trimethylsilyl)methyl, trichloroethoxymethyl, tert-butyldimethylsilyloxymethyl, pivaloyloxymethyl, cyanomethyl, benzyl, 4-methoxybenzyl, 2,4-dimethoxybenzyl, 3,4-dimethoxybenzyl, 2-acetoxy-4-methoxybenzyl, o-nitrobenzyl, bis(4-methoxyphenyl)phenylmethyl, bis(4-methylsulfinylphenyl)methyl, pyrrolidinomethyl, diethoxymethyl, 1-methoxy-2,2-dimethylpropyl or 2-(4-methylsulfonyl)ethyl.

It is a characteristic of protecting groups that they are simple to remove (that is to say without undesirable secondary reactions taking place), for example by solvolysis, reduction, photolysis or alternatively under conditions analogous to physiological conditions, for example enzymatically.

The instant hydrogenation process yields predominantly syn-diols. Depending on the substrate (compound of formula (I)) and the process conditions the syn-diol exceeds, for example, 60%, preferably 70% and most preferred 90%, of the total diol produced.

Suitable solvents are alcohols, especially lower alkanoles such as methanol, ethanol, propanol or butanol, or ethylenglykol, diethylenglykol, ethylenglykolmonomethyl- or monoethylether, diethylenglykolmonomethyl- or monoethyletheror ketones such as acetone or methylisobutylketone. The solvent may also be a mixture of solvents or a mixture of a solvent or solvents with water, for example a mixture of methanol with water.

A suitable ratio by weight of such a catalyst in relation to the substrate is between 1:5 and 1:100, preferably between 1:7 and 1:15.

Heterogeneous platinum catalysts are known per se, are well described in the literature and are commercially available. It is possible to use platinum in the form of the pure metal, for example as a powder, or, what is preferred, in the form of finely distributed metal on a support. A suitable support material is for example carbon, metal oxides like SiO₂, TiO₂, Al₂O₃, metal salts, and natural or synthetic silicates. The catalyst may also be in the form of colloidal platinum. The amount of platinum metal is for example 1 to 10 % by weight, preferably 3 to 8 % by weight, relative to the support.

The hydrogenation is carried out for example with a hydrogen pressure of up to 200 bar, preferably with a hydrogen pressure of 1 to 200 bar and most preferred with a hydrogen pressure of 5 to 40 bar.

The reaction is carried out, for example, at a temperature between 0 and 80°C, especially between 20 to 25°C.

Suitable magnesium salts are the customary salts in hydrated or pure form, for example magnesium acetate, magnesium chloride, magnesium bromide, magnesium ascorbate, magnesium gluconate, magnesium stearate, magnesium nitrate, magnesium sulfate and magnesium citrate whereby magnesium acetate (especially as the tetrahydrate) is particularly preferred.

A suitable ratio by weight of the magnesium salt to the heterogeneous platinum catalyst in the instant process is from 10:1 to 1:10, preferably from 5:1 to 1:5, and most preferred from 5:1 to 1:2, whereby for above calculation purpose the magnesium salt is in the form of magnesium acetate and the heterogeneous catalyst is in the form of a carbon support with 5% by weight of platinum.

A further aspect of the instant invention is the process, wherein a compound of formula (I) 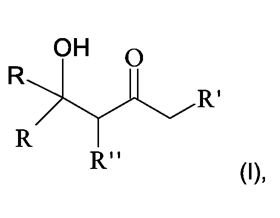 wherein R, R' and R" are independently of each other a radical being compatible with the reaction conditions,
is reduced with hydrogen to the corresponding diol, which is predominantly in the form of the syn-diol, in the presence of a magnesium salt and a heterogeneous platinum catalyst in a solvent, whereby a catalytic amount of an oxidant is added to the reaction mixture. The definitions, preferences and reaction conditions given above are also valid for this aspect of the invention.

Suitable oxidants are, for example, ozone, organic or inorganic peroxides and preferably air, oxygen or H₂O₂ (most preferred 30% H₂O₂ in water), whereby the oxidant is added to a suspension of substrate and catalyst prior to pressurizing with hydrogen. Preferably such catalytic amount is 1 to 100 µl H₂O₂ per 100 mg of substrate used (or the respective molar equivalent in case of a different oxidant).

Compounds of formula (I) are preferred wherein one radical R is H, R" is H and R or R' is -C(=O)OR_{b} and R_{b} is H or a carboxy-protecting group.

Compounds of formula (I) are preferred wherein one radical R is H, R" is H and R is -C(=O)OR_{b}; R' is substituted or unsubstituted alkyl or substituted or unsubstituted aryl and R_{b} is H or a carboxy-protecting group.

Compounds of formula (I) are preferred wherein one radical R is H or lower alkyl and the other radical R is H, alkyl or aryl, R' is -CO₂R₄ , -C(O)NH₂, -C(O)NHR₄ or -C(O)NR₄R₅, R₄ and R₅ areindependently from each other alkyl and R" is H, C₁-C₁₂-alkyl), cycloalkyl or aryl.

Compounds of formula (I) are preferred wherein one radical R is H or lower alkyl and the other radical R is -CH₂CO₂R₄, -CHR₄CO₂R₅, -CO₂R₄, -CH₂C(O)NH₂, -CH₂C(O)NHR₄, -CH₂C(O)NR₄R₅, -CHR₄C(O)NH₂, -CHR₄C(O)NHR_{5,} -CHR₄C(O)NR₅R₆, -C(O)-NH₂, -C(O)-NHR₄ or -C(O)-NR₄R₅;
R' is substituted or unsubstituted alkyl or substituted or unsubstituted aryl;
R₄, R₅ and R₆ are independently from each other alkyl and
R" is H, C₁-C₁₂-alkyl, cycloalkyl or aryl.

### Examples

### H1:

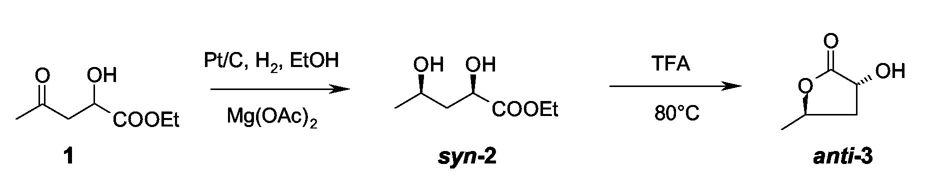

100 mg of 5% Pt/C (Engelhard 4709) and 100 mg of Mg(OAc)₂·4H₂O are placed in a 2.5 ml vial equipped with a small magnetic stirring bar. After the addition of 1 ml of EtOH, 100 µl of substrate **1** are added. The vial is placed in a 50 ml autoclave, the autoclave is sealed, purged with argon (3 times) and with hydrogen (3 times). Then the autoclave is pressurized to 20 bar with hydrogen and the magnetic stirring is started. The reaction is run for 19 h at room temperature (20 to 25 °C). Then the pressure is released, and the autoclave is purged with argon. The product is filtered and evaporated to dryness giving a mixture of **2** and **3** in quantitative yield. This mixture is dissolved in 2 ml of toluene. After the addition of 20 µl of trifluoroacetic acid, the mixture is stirred at 80°C overnight. The reaction mixture is again evaporated to dryness and analyzed by ¹³C NMR. Product identification was performed by comparison with literature data: Blandin, V.; Carpentier, J.-F.; Mortreux A.; *Eur. J. Org. Chem.* **1999,** 1787. Results: Analysis of the ¹³C NMR shows 100% conversion and a 30:70 ratio of *syn:anti* lactone **3,** resulting from a 70:30 *syn:anti* diol mixture.

### H2:

The reaction is carried out as described in H1, but without the addition of the Mg(OAc)₂·4H₂O salt. Results: The analysis of the ¹³C NMR shows 100% conversion and a 43:57 ratio of *syn:anti* lactone **3**, resulting from a 57:43 *syn:anti* diol mixture.

### H3 - H6:

Table 1 lists conversions of several experiments to show the accelerating effect of Mg(OAc)₂·4H₂O. The conditions are identical to the ones described for H1 and H2, except for the changes noted in Table 1.

**Table 1:**

| Example | Catalyst [mg] | Additive | Additive [mg] | time [min] | conv. **1** [%] GC |
|---|---|---|---|---|---|
| H3 | 20 | - | - | 120 | 4 |
| H4 | 20 | Mg(OAc)₂·4H₂O | 5 | 120 | 13 |
| H5 | 100 | - | - | 1260 | 36 |
| H6 | 100 | Mg(OAc)₂·4H₂O | 100 | 1260 | 97 |

### H7:

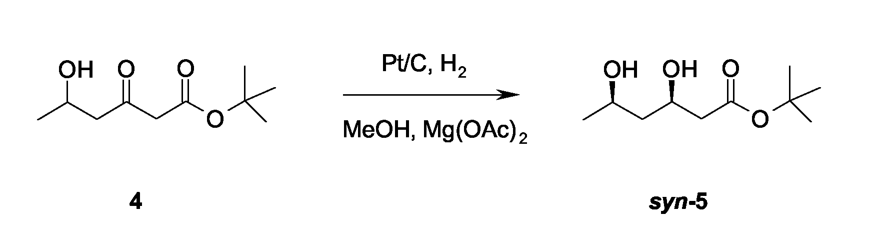

20 mg of 5% Pt/C (Engelhard 4709) and 20 mg of Mg(OAc)₂·4H₂O are placed in a 2.5 ml vial equipped with a small magnetic stirring bar. After the addition of 1 ml of MeOH, 100 µl of substrate **4** are added. The vial is placed in a 50 ml autoclave, the autoclave is sealed, purged with argon (3 times) and with hydrogen (3 times). Then the autoclave is pressurized to 20 bar and the magnetic stirring is started. The reaction is run for 3 h at room temperature (20 to 25 °C). The pressure is released, and the autoclave is purged with argon. The product is filtered and evaporated to dryness. Product identification was performed by comparison with literature data: Masoni, C.; Deschenaux, P. F.; Kallimopoulos, T.; Jacot-Guillarmod, A.; *Helv. Chim. Acta* **1989,** 72, 1284. Conversion and diastereomeric ratio are determined by NMR and GC analysis; **5** is obtained in 83% yield with a *syn:anti* ratio of 6.5.

### H8:

The reaction is conducted as described for H7, but without the addition of the salt. Table 2 compares the results and shows the effect of the magnesium salt additive.

**Table 2:**

| Example | Additive | Additive [mg] | conv. **4** [%] (¹³C NMR) | Syn:anti ratio (¹³C NMR) |
|---|---|---|---|---|
| H7 | Mg(OAc)₂·4H₂O | 20 | 83 | 6.5 |
| H8 | - | - | 18 | 1.6 |

### H9-H13:

Table 3 lists conversions and diastereomeric ratios of several experiments to show the influence of the catalyst type. The conditions are identical to the ones described for H7 except for the changes noted in Table 3.

**Table 3:**

| Example | Catalyst type | conv. **4** [%] (¹³C NMR) | Syn:anti ratio (¹³C NMR) |
|---|---|---|---|
| H9 | 5% Pt/C Typ R (Degussa) | 29 | 5.0 |
| H10 | 5% Pt/C Typ F 101 (Degussa) | >95 | 7.7 |
| H11 | 5%Pt/AloxF214_120(Degussa) | 92 | 7.2 |
| H12 | 5% Ru/C (Engelhard 4857) | 54 | 1.1 |
| H13 | 5% Rh/C (Engelhard 4806) | 82 | 4.6 |

### H14 - H18:

Table 4 lists conversions and diastereomeric ratios of several experiments to show the influence of the type of magnesium salt additive. The conditions are identical to the ones described for H7 except for the changes noted in Table 4.

**Table 4:**

| Example | Additive | Additive [mg] | conv. **4** [%] (¹³C NMR) | Syn:anti ratio (¹³C NMR) |
|---|---|---|---|---|
| H14 | Mg (OOH)₂ | 20 | 33 | 4.8 |
| H15 | Mg(NO₃)₂·6H₂O | 24 | 33 | 6.5 |
| H16 | MgCl₂·6H₂O | 19 | 87 | 7.8 |
| H17 | MgBr₂·6H₂O | 17 | 42 | 7.7 |
| H18 | MgO | 4 | 27 | 5.0 |

### H19-H22:

Table 5 lists conversions and diastereomeric ratios of several experiments to show the influence of the solvent. The conditions are identical to the ones described for H7 except for the changes noted in Table 5.

**Table 5:**

| Example | Solvent [1 ml] | conv. **4** [%] (¹³C NMR) | Syn:anti ratio (¹³C NMR) |
|---|---|---|---|
| H19 | THF | 65 | 4.5 |
| H20 | EtOH | 59 | 4.6 |
| H21 | Toluol | >95 | 3.4 |
| H22 | MeOH:H₂O = 9:1 | 83 | 6.5 |

### H23:

20 mg of 5% Pt/C (Engelhard 4709) and 20 mg of Mg(OAc)₂·4H₂O are placed in a 2.5 ml vial equipped with a small magnetic stirring bar. After the addition of 1 ml of MeOH, 100 µl of substrate **4** and 1 µl of 30% H₂O₂ solution are added. The vial is placed in a 50 ml autoclave, the autoclave is sealed, purged with argon (3 times) and with hydrogen (3 times). Then the autoclave is pressurized to 20 bar with hydrogen and the magnetic stirring is started. The reaction is run for 3 h at room temperature (20 to 25°C). The pressure is released, and the autoclave is purged with argon. The product is filtered and evaporated to dryness. Conversion and diastereomeric ratio are determined by NMR and GC analysis; **5** is obtained in >95% yield with a *syn:anti* ratio of 5.4.

### H24 - H26:

Table 6 lists conversions and diastereomeric ratios of several experiments to show the influence of the H₂O₂ additive. The conditions are identical to the ones described for H19 except for the changes noted in Table 6.

**Table 6:**

| Example | Catalyst [mg] | H₂O₂ [µl] | conv. **4** [%] (¹³C NMR) | Syn:anti ratio (¹³C NMR) |
|---|---|---|---|---|
| H23 | 20 | 1 | >95 | 5.4 |
| H24 | 20 | 10 | >95 | 7.2 |
| H25 | 20 | 100 | >95 | 5.9 |
| H26 | 10 | 10 | 93 | 7.0 |

### H27 - H38:

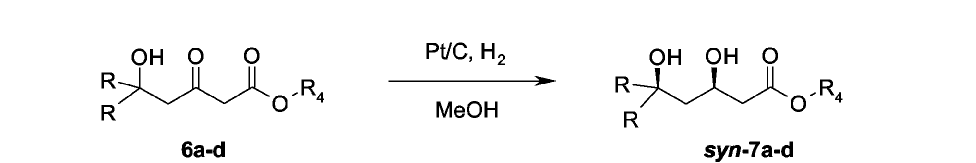

Table 7 lists conversions and diastereomeric ratios of several experiments conducted with substrates **6** differing with respect to substituents R, R' and R" to show the scope of the magnesium salt/H₂O₂ effect. The conditions are identical to the ones described for H19 except for the changes noted in Table 7. Product identification was performed by comparison with literature data: (a) Homma, K.; Takenoshita, H.; Mukaiyama, T.; *Bull. Chem. Soc. Jpn.* **1990**, *63*, 1898. (b) Evans, D. A.; Fitch, D. M.; Smith, T. E.; Cee, V. J.; *J. Am. Chem. Soc.* **2000**, *122*, 10033.

**Table 7:**

| Example | Substrate | R | R | R₄ | Mg(OAc)₂ [mg] | H₂O₂ [µl] | conv. **6** [%] (¹³C NMR) | Syn:anti ratio (¹³C NMR) |
|---|---|---|---|---|---|---|---|---|
| H27 | **6a** | phenyl | H | ethyl | - | - | 35 | 2.3 |
| H28 | **6a** | phenyl | H | ethyl | 20 | - | >95 | 6.1 |
| H29 | **6a** | phenyl | H | ethyl | 20 | 10 | >95 | 6.7 |
| H30 | **6b** | phenyl | methyl | ethyl | - | - | 7 | 3.0 |
| H31 | **6b** | phenyl | methyl | ethyl | 20 | - | 22 | 6.7 |
| H32 | **6b** | phenyl | methyl | ethyl | 20 | 10 | 71 | 8.4 |
| H33 | **6c** | phenyl | H | tert-butyl | - | - | 28 | 2.0 |
| H34 | **6c** | phenyl | H | tert-butyl | 20 | - | 77 | 4.9 |
| H35 | **6c** | phenyl | H | tert-butyl | 20 | 10 | >95 | 4.7 |
| H36 | **6d** | CH₂OCH₂Ph | H | tert-butyl | - | - | 13 | 1.6 |
| H37 | **6d** | CH₂OCH₂Ph | H | tert-butyl | 20 | - | 57 | 5.1 |
| H38 | **6d** | CH₂OCH₂Ph | H | tert-butyl | 20 | 10 | 95 | 5.9 |

### H39 - H44:

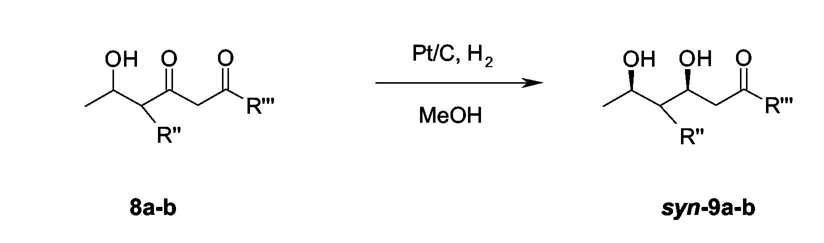

Table 8 lists conversions and diastereomeric ratios of several experiments conducted with substrates differing with respect to substituents R" and R"' to show the scope of the magnesium salt/H₂O₂ effect. The conditions are identical to the ones described for H19 except for the changes noted in Table 8. Product identification was performed by comparison with literature data: Evans, D. A.; Chapman, K. T.; Carreira, E. M. *J. Am. Chem. Soc.* **1988,** *110,* 3560.

**Table 8:**

| Example | Substrate | R" | R"' | Mg(OAc)₂ [mg] | H₂O₂ [µl] | conv. 8 [%] (¹³C NMR) | Syn:anti ratio (¹³C NMR) |
|---|---|---|---|---|---|---|---|
| H39 | **8a** | methyl | -O-tert-butyl | - | - | <5 | - |
| H40 | **8a** | methyl | -O-tert-butyl | 20 | - | >95 | 4.0^{a} |
| H41 | **8a** | methyl | -O-tert-butyl | 20 | 1 | >95 | 3.9^{a} |
| H42 | **8b** | H | NEt₂ | - | - | 35 | 1.6 |
| H43 | **8b** | H | NEt₂ | 20 | - | >95 | 5.0 |
| H44 | **8b** | H | NEt₂ | 20 | 10 | >95 | 4.9 |

| | | | | | | | |
|---|---|---|---|---|---|---|---|
| ^{a} Ratio between sum of both *syn-* and sum of both *anti*-isomers, assuming the *syn*-isomers to be formed in excess. | | | | | | | |

### Experimental details:

Hydrogenation reactions were performed with p.a. grade solvents in a 50 ml stainless steel autoclave positioned on a magnetic stirrer. In a typical screening run, the autoclave was loaded with four 2.5 ml glass vials to conduct four different experiments at the same time, temperature and pressure.
Commercially available compounds needed as substrates or for substrate synthesis were purchased from Fluka or Aldrich Chemicals and used without further purification. Compounds **4, 6a-d** and **8a-b** were synthesized by aldol type condensations of β-ketoesters or -amides and the respective aldehydes or ketones: Huckin, S. N.; Weiler, L.; *Can. J. Chem.* **1974,** *22*, 2157. Commercially unavailable β-ketoesters were prepared by the reaction of Meldrum's acid with the respective acid chloride: Oikawa, Y.; Sugano, K.; Yonemitsu O.; *J. Org. Chem.* **1978,** *10,* 2087. The stereochemical assignment of *syn* and *anti* descriptors for product 9b was confirmed by preferential reaction of the *syn*-diols (especially the minor) of the product mixture into the respective para-methoxybenzylidene acetals, whereas the *anti*-diols were largely left unaffected by the reaction conditions: Evans, D. A.; Ng, H. P. *Tetrahedron Lett.* **1993**, *34*, 2229.
NMR measurements were performed on a Bruker 300 MHz apparatus; GC analyses were conducted with a Carlo Erba GC 6000 equipped with a β-Dex 110 column 30 m x 0.25 mm (Supelco); H₂ was used as the carrier gas. Injector temperature: 220 °C. Detector temperature: 250 °C.

### Analysis data for compounds 3; 5, 7a-d. 9a-b:

### Anti-5-hydroxy-3-methyl-tetrahydrofuran-2-one, anti-3:

¹H-NMR (300.13 MHz; CDCl₃): δ 1.38 (d, CH₃, *J* = 7.8 Hz); 2.12 (m, C*H*H), 2.36 (m, CH*H*); 4.52 (m, CH); 4.79 (m, CH) ppm.
¹³C-NMR: (75.47 MHz; CDCl₃): δ 21.43 (CH₃); 37.07 (CH₂); 67.53 (CH); 75.00 (CH); 177.59 (C=O) ppm.
GC: Temperature program: 105 °C: 20 min, 10 °C / min to 180 °C; 180 °C: 2.5 min. t_{R} = 19.51, 19.86 min.

### Syn-5-hydroxy-3-methyl-tetrahydrofuran-2-one, syn-3:

¹H-NMR (300.13 MHz; CDCl₃): δ 1.45 (d, CH₃, *J* = 7.4 Hz); 1.87 (m, C*H*H), 2.74 (m, CH*H*); 4.43 (m, CH); 4.76 (m, CH) ppm.
¹³C-NMR: (75.47 MHz; CDCl₃): δ 20.88 (CH₃); 38.78 (CH₂); 69.02 (CH); 73.64 (CH); 177.93 (C=O) ppm.
GC: t_{R} = 15.22, 15.98 min.

### Syn-3,5-dihydroxyhexanoic acid t-butyl ester, syn-5:

¹H-NMR (300.13 MHz; CDCl₃): δ 1.20 (d, CH₃, *J* = 6.2 Hz); 1.46 (s, C(CH₃)₃); 1.53 (m, CH₂); 2.40 (d, CH₂, *J* = 6.3 Hz); 3.47 (s, OH); 4.08 (m, CH); 4.22 (m, CH) ppm.
¹³C-NMR: (75.47 MHz; CDCl₃): δ 24.13 (CH₃); 28.42 (C(CH₃)₃); 43.16 (CH₂); 44.22 (CH₂); 68.49 (CH); 69.37 (CH); 81.80 (C); 172.36 (C=O) ppm.
GC: Temperature program: 150 °C: 5 min, 1 °C / min to 160 °C; 160 °C: 1 min, 15 °C / min to 220 °C, 220 °C: 1 min. t_{R} = 6.58 min.

### Anti-3,5-dihydroxyhexanoic acid t-butyl ester, anti-5:

¹H-NMR (300.13 MHz; CDCl₃): δ 1.23 (d, CH₃, *J* = 6.2 Hz); 1.46 (s, C(CH₃)₃); 1.55 (m, CH₂); 2.40 (d, CH₂, *J* = 6.3 Hz); 3.47 (s, OH); 4.10 (m, CH); 4.24 (m, CH) ppm.
¹³C-NMR: (75.47 MHz; CDCl₃): δ 24.01 (CH₃); 28.42 (C(CH₃)₃); 42.65 (CH₂); 43.99 (CH₂); 65.22 (CH); 66.10 (CH); 81.69 (C); 172.69 (C=O) ppm.
GC: t_{R} = 6.79 min.

### Syn-3,5-dihydroxy-5-phenyl-pentanoic acid ethyl ester, syn-7a:

¹H-NMR (300.13 MHz; CDCl₃): δ 1.24 (t, CH₃, *J* = 5.8 Hz); 1.75-2.00 (m, CH₂, 2 OH); 2.22 (m, CH₂); 4.16 (t, C*H*₂CH₃, *J* = 5.7 Hz); 4.34 (m, CH); 4.99 (dd, CH, *J* = 3.3, 9.6 Hz); 7.27-7.39 (m, 5 Ph-H) ppm.
¹³C-NMR: (75.47 MHz; CDCl₃): δ 14.53 (CH₃); 42.01 (CH₂); 45.15 (CH₂); 61.21 (CH₃CH₂); 68.99 (CH); 74.74 (CH); 126.12 (Ph-CH); 127.97 (Ph-CH); 128.85 (Ph-CH); 144.52 (Ph-C); 172.87 (C=O) ppm.

### Anti-3,5-dihydroxy-5-phenyl-pentanoic acid ethyl ester, anti-7a:

¹H-NMR (300.13 MHz; CDCl₃): δ 1.22 (t, CH₃, *J* = 5.8 Hz); 1.75-2.00 (m, CH₂, 2 OH); 2.24 (m, CH₂); 4.14 (q, C*H*₂CH₃, *J* = 5.7 Hz); 4.34 (m, CH); 5.03 (dd, CH, *J* = 3.2, 9.5 Hz); 7.28-7.38 (m, 5 Ph-H) ppm.
¹³C-NMR: (75.47 MHz; CDCl₃): δ 14.53 (CH₃); 41.59 (CH₂); 44.54 (CH₂); 61.21 (CH₃CH₂); 65.89 (CH); 71.48 (CH); 125.93 (Ph-CH); 127.69 (Ph-CH); 128.87 (Ph-CH); 144.78 (Ph-C); 173.18 (C=O) ppm.

### Syn-3,5-dihydroxy-5-phenyl-hexanoic acid ethyl ester, syn-7b:

¹H-NMR (300.13 MHz; CDCl₃): δ 1.27 (t, CH₂C*H*₃, *J* = 7.2 Hz); 1.68 (s, CH₃); 1.91 (m, CH₂); 2.45 (m, CH₂); 3.85 (d, br, 2 OH; *J* = 7.8 Hz); 4.17 (q, C*H*₂CH₃, *J* = 7.2 Hz); 4.52 (m, CH); 7.23-7.48 (m, 5 Ph-H) ppm.
¹³C-NMR: (75.47 MHz; CDCl₃): δ 14.47 (CH₃CH₂); 28.51 (CH₃); 42.15 (CH₂); 48.89 (CH₂); 61.18 (CH₃CH₂); 66.63 (CH); 74.80 (C); 124.80 (Ph-CH); 127.22 (Ph-CH); 128.60 (Ph-CH); 149.23 (Ph-C); 172.89 (C=O) ppm.

### Anti-3,5-dihydroxy-5-phenyl-hexanoic acid ethyl ester, anti-7b:

¹H-NMR (300.13 MHz; CDCl₃): δ 1.18 (t, CH₂C*H*₃, *J* = 5.7 Hz); 1.50 (s, CH₃); 1.98 (m, CH₂); 2.40 (m, CH₂); 3.85 (d, br, 2 OH; *J* = 7.8 Hz); 4.14 (q, C*H*₂CH₃, *J* = 7.2 Hz); 4.52 (m, CH); 7.23-7.48 (m, 5 Ph-H) ppm.
¹³C-NMR: (75.47 MHz; CDCl₃): δ 14.47 (CH₃CH₂); 30.89 (CH₃); 41.90 (CH₂); 47.80 (CH₂); 61.18 (CH₃CH₂); 66.88 (CH); 73.72 (C); 124.68 (Ph-CH); 127.04 (Ph-CH); 128.73 (Ph-CH); 147.61 (Ph-C); 172.82 (C=O) ppm.

### Syn-3,5-dihydroxy-5-phenyl-pentanoic acid tert-butyl ester, syn-7c:

¹H-NMR (300.13 MHz; CDCl₃): δ 1.46 (s, C(CH₃)₃); 1.70-1.76 (m, CH₂); 2.41 (m, CH₂); 3.62 (s, br, OH); 3.84 (s, br, OH); 4.30 (m CH); 4.97 (dd, CH, *J* = 3.0, 9.6 Hz); 7.28-7.39 (m, 5 Ph-H) ppm.
¹³C-NMR: (75.47 MHz; CDCl₃): δ 28.30 (C(CH₃)₃); 43.09 (CH₂); 45.13 (CH₂); 69.01 (CH); 74.58 (CH); 81.83 (C); 126.00 (Ph-CH); 127.87 (Ph-CH); 128.79 (Ph-CH); 144.59 (Ph-C); 172.28 (C=O) ppm.

### Anti-3,5-dihydroxy-5-phenyl-pentanoic acid tert-butyl ester, anti-7c:

¹H-NMR (300.13 MHz; CDCl₃): δ 1.45 (s, C(CH₃)₃); 1.76-2.00 (m, CH₂); 2.43 (m, CH₂); 3.62 (s, br, OH); 3.84 (s, br, OH); 4.26 (m CH); 5.04 (m, CH); 7.27-7.35 (m, 5 Ph-H) ppm.
¹³C-NMR: (75.47 MHz; CDCl₃): δ 28.30 (C(CH₃)₃); 42.69 (CH₂); 44.62 (CH₂); 65.98 (CH); 71.31 (CH); 81.83 (C); 125.92 (Ph-CH); 127.57 (Ph-CH); 128.75 (Ph-CH); 144.92 (Ph-C); 172.61 (C=O) ppm.

### Syn-3,5-dihydroxy-6-benzyloxy-hexanoic acid tert-butyl ester, syn-7d:

¹H-NMR (300.13 MHz; CDCl₃): δ 1.46 (s, C(CH₃)₃); 1.63 (m, CH₂); 2.41 (m, CH₂); 3.31 (s, br, OH); 3.45 (m, CH₂); 3.80 (s, br, OH); 4.08 (m, CH); 4.21 (m, CH); 4.56 (s, Ph-CH₂); 7.29-7.35 (m, 5 Ph-H) ppm.
¹³C-NMR: (75.47 MHz; CDCl₃): δ 28.09 (C(CH₃)₃); 38.89 (CH₂); 42.61 (CH₂); 68.25 (CH); 70.41 (CH); 73.39 (CH₂); 74.16 (CH₂); 81.33 (C); 127.77 (Ph-CH); 127.96 (Ph-CH); 128.45 (Ph-CH); 137.93 (Ph-C); 171.94 (C=O) ppm.

### Anti-3,5-dihydroxy-6-benzyloxy-hexanoic acid tert-butyl ester, anti-7d:

¹H-NMR (300.13 MHz; CDCl₃): δ 1.46 (s, C(CH₃)₃); 1.65 (m, CH₂); 2.35 (m, CH₂); 3.27 (s, br, OH); 3.45 (m, CH₂); 3.80 (s, br, OH); 4.06 (m, CH); 4.23 (m, CH); 4.56 (s, Ph-CH₂); 7.27-7.34 (m, 5 Ph-H) ppm.
¹³C-NMR: (75.47 MHz; CDCl₃): δ 28.09 (C(CH₃)₃); 38.79 (CH₂); 42.50 (CH₂); 65.44 (CH); 67.56 (CH); 73.32 (CH₂); 74.38 (CH₂); 81.33 (C); 127.96 (Ph-CH); 128.02 (Ph-CH); 128.27 (Ph-CH); 137.93 (Ph-C); 172.24 (C=O) ppm.

### Major syn-3,5-dihydroxy-4-methyl-hexanoic acid tert-butyl ester, major syn-9a:

¹H-NMR (300.13 MHz; CDCl₃): δ 0.95 (d, CH₃, *J* = 7.2 Hz); 1.18 (d, CH₃, *J* = 6.3 Hz ); 1.40 (m, CH₂); 1.47 (s, C(CH₃)₃); 2.01 (s, br, 2 OH); 2.33 (m, C*H*H); 2.47 (m, CH*H*); 4.14 (m, CH); 4.24 (m, CH) ppm.
¹³C-NMR: (75.47 MHz; CDCl₃): δ 5.24 (CH₃); 21.45 (CH₃); 28.44 (C(CH₃)₃); 40.82 (CH₂); 42.59 (CH); 72.11 (CH); 73.04 (CH); 81.70 (C(CH₃)₃); 172.93 (C=O) ppm.

### Minor syn-3,5-dihydroxy-4-methyl-hexanoic acid tert-butyl ester, minor syn-9a:

¹H-NMR (300.13 MHz; CDCl₃): δ 0.79 (d, CH₃, *J* = 6.9 Hz); 1.20 (d, CH₃, *J* = 6.6 Hz ); 1.46 (s, C(CH₃)₃); 1.55 (m, CH₂); 2.01 (s, br, 2 OH); 2.36 (m, C*H*H); 2.51 (m, CH*H*); 3.82 (m, CH); 3.95 (m, CH) ppm.
¹³C-NMR: (75.47 MHz; CDCl₃): δ 13.01 (CH₃); 21.45 (CH₃); 28.32 (C(CH₃)₃); 40.62 (CH₂); 45.29 (CH); 72.03 (CH); 77.86 (CH); 81.90 (C(CH₃)₃); 173.12 (C=O) ppm.

### Syn-3,5-dihydroxyhexanoic acid diethyl amide, syn-9b:

¹H-NMR (300.13 MHz; CDCl₃): δ 1.20 (m, 3 CH₃); 1.63 (m, CH₂); 2.44 (m, CH₂); 3.25 (m, CH₂CH₃); 3.35 (m, C*H*₂CH₃); 3.44 (s, br, 2 OH); 4.06 (m, CH); 4.26 (m, CH) ppm.
¹³C-NMR: (75.47 MHz; CDCl₃): δ 13.22 (CH₂CH₃); 14.32 (CH₂CH₃); 23.79 (CH₃); 39.70 (CH₂); 40.63 (CH₂); 42.40 (CH₂); 44.37 (CH₂); 68.13 (CH); 69.31 (CH); 171.86 (C=O) ppm.

GC: Temperature program: 120 °C: 5 min, 1 °C / min to 160 °C; 160 °C: 10 min. t_{R} = 39.03 min.

### Anti-3,5-dihydroxyhexanoic acid diethyl amide, anti-9b:

¹H-NMR (300.13 MHz; CDCl₃): δ 1.23 (m, 3 CH₃); 1.65 (m, CH₂); 2.40 (m, CH₂); 3.25 (m, C*H*₂CH₃); 3.35 (m, C*H*₂CH₃); 3.44 (s, br, 2 OH); 4.12 (m, CH); 4.32 (m, CH) ppm.
¹³C-NMR: (75.47 MHz; CDCl₃): δ 13.22 (CH₂CH₃); 14.32 (CH₂CH₃); 24.00 (CH₃); 39.03 (CH₂); 40.74 (CH₂); 42.51 (CH₂); 45.16 (CH₂); 64.77 (CH); 66.08 (CH); 172.37 (C=O) ppm.
GC: t_{R} = 39.30 min.

## Claims

1. A process, wherein a compound of formula (I) 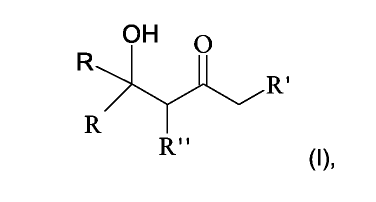 wherein R, R' and R" are independently of each other a radical being compatible with the reaction conditions, except compounds wherein
a) one R is H and the other R is -CH₂CN, R" is H and R' is -C(=O)OR_{b} and R_{b} is a H or a carboxy-protecting group;
b) one R is H and the other R is -CH₂C(=O)NR*R**, R" is H and R' is -C(=O)OR_{b} and R* and R** are independently of each other H or an amide-protecting group and R_{b} is H or a carboxy-protecting group;
c) one R is H and the other R is -CH₂C(=O)OR_{b}, R" is H and R' is -CH₂-N₃ and R_{b} is H or a carboxy-protecting group; and
d) one R is H and the other R is -CH₂C(=O)OR_{b}, R" is H and R' is -CH₂-R_{d} and R_{b} is H or a carboxy-protecting group and R_{d} is halogen;
is reduced with hydrogen to the corresponding diol, which is predominantly in the form of the syn-diol, in the presence of a magnesium salt and a heterogeneous platinum catalyst in a solvent.

2. A process according to claim 1, wherein the magnesium salt is magnesium acetate tetrahydrate.

3. A process according to claim 1 wherein the support material for the heterogeneous platinum catalyst is selected from carbon or Al₂O₃.

4. A process according to claim 1 wherein the solvent is an alcohol or a mixture of an alcohol with water.

5. A process according to claim 1 wherein for the compound of formula (I) at least one radical R is H or lower alkyl.

6. A process according to claim 1 wherein for the compound of formula (I) one radical R is H or lower alkyl and the other radical R is H, alkyl or aryl, R' is -CO₂R₄, -C(O)NH₂, -C(O)NHR₄ or -C(O)NR₄R₅, R₄ and R₅ is independently from each other alkyl and R" is H, C₁-C₁₂-alkyl), cycloalkyl or aryl.

7. A process according to claim 1 wherein for the compound of formula (I) one radical R is H or lower alkyl and the other radical R is -CH₂CO₂R₄, -CHR₄CO₂R₅, -CO₂R₄, -CH₂C(O)NH₂, -CH₂C(O)NHR₄, -CH₂C(O)NR₄R₅, -CHR₄C(O)NH₂, -CHR₄C(O)NHR₅, -CHR₄C(O)NR₅R₆, -C(O)-NH₂, -C(O)-NHR₄ or -C(O)-NR₄R₅;
R' is H, substituted or unsubstituted alkyl or substituted or unsubstituted aryl;
R₄, R₅ and R₆ are independently from each other alkyl and
R" is H, C₁-C₁₂-alkyl, cycloalkyl or aryl.

8. A process, wherein a compound of formula (I) 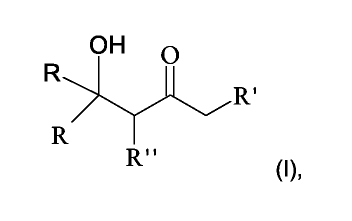 wherein R, R' and R" are independently of each other a radical being compatible with the reaction conditions,
is reduced with hydrogen to the corresponding diol, which is predominantly in the form of the syn-diol, in the presence of a magnesium salt and a heterogeneous platinum catalyst in a solvent, whereby a catalytic amount of an oxidant is added to the reaction mixture.

9. A process according to claim 8 wherein the oxidant is H₂O₂ in water.

10. A process according to claim 8 wherein for the compound of formula (I) one radical R is H or lower alkyl and the other radical R is H, alkyl or aryl, R' is -CO₂R₄, -C(O)NH₂, -C(O)NHR₄ or -C(O)NR₄R₅, R₄ and R₅ is independently from each other alkyl and R" is H, C₁-C₁₂-alkyl), cycloalkyl or aryl.
